# EUROPEAN PATENT SPECIFICATION

(11) **EP 2 125 059 B1**
(45) Date of publication and mention of the grant of the patent: **13.08.2014**
(21) Application number: 07835185.5
(22) Date of filing: 06.11.2007
(51) Int. Cl.: A61L 27/54, A61P 29/00, A61L 27/06, A61K 9/16, A61K 9/50

(54) **TREATMENT OF INFLAMMATORY AND/OR BACTERIAL CONDITIONS WITH PARTICLES OF MICROSTRUCTURE**
BEHANDLUNG VON ENTZÜNDLICHEN UND/ODER BAKTERIELLEN ERKRANKUNGEN MIT TEILCHEN MIT MIKROSTRUKTUR
TRAITEMENT DE PATHOLOGIES INFLAMMATOIRES ET/OU BACTÉRIENNES AU MOYEN DE PARTICULES DE MICROSTRUCTURE

(30) Priority: 22.02.2007 SE 0700456; 22.02.2007 US 902501 P
(43) Date of publication of application: 02.12.2009
(73) Proprietor: Tigran Technologies AB (publ), 205 12 Malmö (SE)
(72) Inventor: BJURSTEN, Lars, Magnus, S-216 11 Limhamn (SE); MJÖBERG, Bengt, S-271 35 Ystad (SE)
(74) Representative: Stenbäck, Maria Elisabeth
(86) International application number: PCT/SE2007/000985
(87) International publication number: WO 2008/103082

(56) References cited:
- EP-A1- 0 856 299
- WO-A1-89/06548
- WO-A1-98/51231
- WO-A1-2006/126312
- WO-A2-00/64504
- US-A- 5 015 256
- US-A- 5 045 318
- US-A1- 2005 031 663
- US-A1- 2005 158 393
- US-A1- 2006 161 256
- US-A1- 2007 003 752
- CAI R ET AL: "INDUCTION OF CYTOTOXICITY BY PHOTOEXCITED TITANIUM DIOXIDE PARTICLES", CANCER RESEARCH, AMERICAN ASSOCIATION FOR CANCER RESEARCH, US, vol. 52, no. 8, 15 April 1992 (1992-04-15) , pages 2346-2348, XP009151896, ISSN: 0008-5472

## Description

### Field of the invention

The present invention refers to use of an injectable suspension comprising particles of microstructure for use as a medicament in the treatment of an inflammatory and/or bacterial condition, such as periodontitis, periimplantitis, and osteitis.

### Background of the invention

It is known that operations and wounds in the body often brings about inflammation and/or infections, which is the case also in connection with implantations, especially in connection with bone tissue, e.g. hip joints and dental applications.

When titanium is exposed to air or water, an oxide layer is spontaneously formed. This spontaneously formed oxide layer is 4-10 nm thick and consists predominantly of TiO₂, Ti(IV), with smaller amounts of Ti(III) and Ti(II) present in the oxide (se references 1, 3 and 4).

The anti-inflammatory and antibacterial effects of titanium are based on the chemical properties of TiO₂ at its surface and may work in several different ways, all related to the exposed surface area. As previously shown (reference 2), TiO₂ has the ability to directly scavenge ROS (reactive oxygen species). One possible mechanism is through a set of catalytic redox reactions that has been suggested for the breakdown of hydrogen peroxide, superoxide and peroxynitrite on titanium dioxide surfaces (references 2 and 5):

2TiO₂ + 2O₂⁻+ 2H⁺→ Ti₂O₃ + 2O₂ + H₂O (1a)

2TiO₂ + H₂O₂ → Ti₂O₃ + O₂ + H₂O (1b)

Ti₂O₃ + OONO⁻ → 2TiO₂ + NO₂⁻ (2a)

Ti₂O₃ + H₂O₂ → 2TiO₂ + H₂O (2b)

Of special interest with respect to the antibacterial effects of titanium is - the possibility that TiO₂ may also react directly with H₂O₂ and form a Ti-peroxy gel, TiOOH(H₂O)ₙ, on the oxide surface. ESR (electron spin resonance) measurements have also shown that superoxide radicals are present in the Ti-peroxy gel, indicating either trapping of superoxide in the gel or direct reaction between superoxide and TI(IV) in the Ti-peroxy gel (references 5-7). Complexes similar to the Ti-peroxy gel might also be formed between TiO₂ and peroxynitrite. It was recently shown that peroxynitrous acid, the protonated form of peroxynitrite (pKₐ = 6.8), forms a complex similar to the Ti-peroxy gel with Ti(IV) under acidic conditions (reference 8). Moreover, the blue tint sometimes found in tissue surrounding titanium implants suggests that Ti(IV) reacts with ROS and forms stable Ti(III) complexes (see reference 9). It has also been shown that the thickness of the titanium oxide layer on implants increases with time in vivo (reference 10), suggesting that Ti metal might act as a sink for oxygen species. All of these reactions might be involved in the direct breakdown of ROS that occurs on the TiO₂ surface and the linked anti-inflammatory effect.

Titanium (that is titanium metal with a surface layer of titanium oxide) has been reported to reduce inflammation (Overgaard, Danielsen et al. 1998) and also to be less susceptible to infections than other materials (Johansson, Lindgren et al. 1999). There are also reports describing unique properties of titanium due to its chemical interactions with reactive oxygen species (ROS). The catalytic property of titanium has been shown to be related to the titanium oxide on the surface being present on surfaces composed of only titanium oxide (Sahlin 2006 et al). Such a catalytic property is e.g. described in the US patent application No. 2005074602 to Bjursten et al and also in the generation of titanium peroxy compounds (Tengvall, Elwing et al. 1989; Tengvall, Lundstrom et al. 1989) with anti-inflammatory (Larsson, Persson et al. 2004) and bactericidal properties (Tengvall, Hornsten et al. 1990). The above beneficial properties of titanium seems thus to be linked to its chemical interaction with a living tissue environment.

Cai et al disclose in "Induction of Cytotoxicity by Photoexcited TiO2 Particles", Cancer Research 52, 2346-2348, April 15, 1992, the use of Ti02 particles in 0.4 ml PBS (1 mg TiO₂/ml) containing 5 % fetal calf serum for injection into a tumour and surrounding subcutaneous tissue.

US 2005/0158393 discloses injection of a suspension comprising a fluid and particles with dimensions of 1 nm to 0.001 mm, wherein the particles may comprise titanium for embolization, dermal augmentation and tissue building, drug delivery, gene therapy, and other prophylactic, therapeutic or cosmetic medical applications.

US 5 045 318 discloses the use of a gel including a titanium peroxy radical and titanium peroxide, together with titanium hydroxide as an anti-inflammatory oxidizing agent.

For references of implants where titanium could be used, US 5,015,256 (Bruce et al) discloses means and a method for fixing an elongate prosthesis, such as the stem of a femoral prosthesis, to living tissue which defines a cavity in which a length of the prosthesis is received with a gap to the boundary of the cavity. Essentially the entire gap is filled with loose, but packed grains of a biocompatible material, said grains interlocking. As an example of granular material titanium is mentioned, and the grains are stated to be irregular, essentially non-elastic and preferably porous, the latter property being said to promote growth of bone tissue which has grown from the osseous wall. The grain interlocking has been achieved by vibrating the stem into a bed of grains housed in said cavity and by a final blow on the stem.

WO00/64504 (Bruce et al) discloses a biocompatible, plastic or essentially non-elastic, porous body, such as a grain, with continuous porosity, the openings of cavities and the passages interconnecting them having a width of > about 50 µm for bone tissue. The term "continuous" is said to mean a porosity which allows bone tissue to grow through the porous body. The porous body may be of titanium.

One disadvantage with the inventions according to US 5,015,256 and WO00/64504 is the fact that the grains according to these inventions are not optimised for anti-inflammatory and/or antibacterial effects. In fact, US 5,015,256 and WO00/64504 do not disclose anything about any possible anti-inflammatory and/or antibacterial effects of the grains or granules disclosed therein. The application areas for the grains according to US 5,015,256 and WO00/64504 are as orthopaedic means, e.g. in a body cavity around a "whole-body" prosthesis for increasing fixation and bone ingrowth, and in dental applications, and in that case also for increasing the ingrowth of bone tissue and e.g. stability and fixation of a dental implant, such as a titanium screw. This "whole-body implant or prosthesis" according to US 5,015,256 and WO00/64504 is as mentioned e.g. a titanium screw or a tooth, and common for all of these are that they have at least one fastening or fixing element. As understandable, the grains according to US 5,015,256 and WO00/64504 surrounding such a "whole-body" prosthesis are not optimised in relation to exhibiting anti-inflammatory and/or antibacterial effects due to the fact that this is not a main focus for these application areas. The structure and size of these grains or granules, such as the high porosity, are optimised for providing enhanced bone ingrowth, and not anti-inflammatory and/or antibacterial effects, especially not these effects in relation to specific inflammatory and bacterial conditions where there might be practical problems on how to reach a typical infected site of the condition.

The present invention aims at solving these problems by providing a method for treating such conditions effectively with respect to enhanced anti-inflammatory and antibacterial effects and practical beneficial purposes.

### Summary of the invention

The problems mentioned above are solved by the resent invention which provides an injectable suspension comprising particles of microstructure comprising titanium, titanium alloy, at least one titanium oxide or a combination thereof, and having a surface with at least a substantial part consisting of at least one type of titanium oxide wherein the particles of microstructure have an average length from one side to the opposite side, through a geometrical centre, of < 1mm; and a fluid vehicle, are brought into contact with at least one infected site in a human or animal body by insertion, injection or implantation, which at least one infected site exhibits the inflammatory and/or bacterial condition.

Some of the important differences of the particles for use according to the present invention are the geometrical structure, size and appearance per se of these in comparison to the grains or granules disclosed in US 5,015,256 and WO00/64504. The particle for use according to the invention are of microstructure, that is of very small size, which means that they are very fine, rendering a high specific surface area. Moreover, the microstructure of these particles is the main reason for them bein very easy to apply on and at different sites. In other words, the particles for use according to the invention appear like fine sand, which should be compared to the porous grains or granules according to US 5,015,256 and WO00/64504. This means that a variety of particles accor-ding to the invention will appear and behave like a powder, which is not the case of a variety of grains according to US 5,015,256 and WO00/64504, which grains would more look like a group of metal stones or corals.

Due to this fact, the utility areas for these different structures are also totally different. As mentioned, the grains or granules according to US 5,015,256 and WO00/64504 are optimised for bone ingrowth, e.g. when filled in a body cavity around a prosthesis. Due to the size of these grains they give stability for such applications, creating a bed of grains. Moreover, the porosity of the grains according to WO00/64504, wherein the openings of cavities and the passages interconnecting them having a width of > about 50 µm for bone tissue, yields enhanced bone ingrowth effect. This minimal size of some of the pores clearly show that this geometrical structure of the grains is different from particles of microstructure, especially in relation to size, but also with respect to the overall appearance.

In the sense of applications for supporting a "whole-body" prosthesis, such as a titanium screw for orthopaedic purposes, the particles of microstructure according to the present invention would not be effective since a bed of these would not give a rigid structure around such a prosthesis. This is, however, not one of the objects according to the invention. Due to the small size of the particles, they each maintain a large specific surface area, which is one of the most important parameters in relation to the anti-inflammatory and antibacterial effects. A high value of the specific surface area yields high anti-inflammatory and antibacterial effects. Moreover, the size of these particles also have practical benefits. Due to the size they can be brought into contact with infected sites through out a human or animal body. Even if one unexpectedly would try to use grains, such as those according to US 5,015,256 and WO00/64504, for the same purpose, they would not be possible to use in the same extent or with the same result. This is due to the fact that they are not of microstructure, and hence are too large for some injection applications. In addition, these grains do not exhibit high values of anti-inflammatory and antibacterial effects, and hence the use disclosed according to the present invention, is not possible to perform with the grains according to any of US 5,015,256 and WO00/64504.

### Detailed description of the invention

Specific embodiments according to the present invention will be described in more detail below. These embodiments should be regarded just as such specific ones, and should not be interpreted as a limitation of the present invention. The scope of the invention is defined by the appended claims.

The material of the particles is essential in relation to the present invention. Firstly it is the matter of the possible compositions of the particles, where titanium is an element always being present. However, it is important to understand that the base metal titanium can be present in a particle for use according to the present invention as an alloy, as pure metal titanium, that is with only possible small amounts of impurities, as a titanium oxide, or a combination thereof. The possible small amounts of impurities in pure titanium are normally oxides or some metals, but could also consist of other chemicals. Moreover, titanium oxide is always present in some extent on the surface of the particles. Different types of possible titanium oxides are disclosed above. Moreover, the mechanisms driving oxide formation on the surface are also described.

Secondly, the geometrical structure and size are important, which has been mentioned above with reference to the expression "microstructure". Therefore, according to one specific embodiment of the present invention, the particles of microstructure for use according to the invention have an average length from one side to the opposite side, through a geometrical centre, of < 1 mm. More specific, the particles of microstructure for use according to the invention have an average length from one side to the opposite side, through a geometrical centre, of < 0.5 mm, even more specific of < 0.2 mm and still more specific of < 0.1 mm. According to one specific embodiment of the present invention, the particles of microstructure for use according to the invention have an average length from one side to the opposite side, through a geometrical centre, in the range of 0.01-0.1 mm, more specific an average length from one side to the opposite side, through a geometrical centre, of < 0.01 mm.

Another important factor in relation to geometrical structure and appearance is the actual geometrical shape of the particles. According to one specific embodiment of the present invention the particles of microstructure for use are in the shape of spheres, spikes, flakes, chips or similar or combinations thereof. The microstructure as well as the shape are parameters affecting the specific surface area, which hence is a direct measure on the specific surface area and therefore the anti-inflammatory and antibacterial effects. In comparison to the grains disclosed according to US 5,015,256 and WO00/64504, all of these different parameters differ with the particles for use according to the present invention, that is size, shape and anti-inflammatory and antibacterial properties.

As mentioned above, there are also practical advantages with utilising particles according to the present invention as a medicament for an inflammatory and/or bacterial condition present in a human or animal body. Due to the fact of the small size of the particles, these could easily be brought into contact with an infected site present in the human or animal body at totally different places in comparison. Specific examples are infected sites in the mouth or close to the teeth, that is for dental applications, but also e.g. in the intestine or totally other different vital organs or tissues. An important example is bone tissue.

For some of these specific applications it is important to provide the particles in some vehicle with the ability to transport the particles to the intended infected site or sites. Therefore, according to the present invention, the particles according to above are mixed with a fluid vehicle to produce an injectable suspension before, or at the same time as the insertion, injection or implantation in the human or animal body. Possible specific fluid vehicles are e.g. NaCl (aq), hyaluronic acid, PEG, propylene glycole alginate (PGA), titanium peroxy gel, methyl cellulose, carbomethyl cellulose, dextran, a high viscous polymeric gel, and a protein solution, or a combination thereof. One example of a protein solution is one possible of serving as a fluid vehicle, such as albumin. According to another specific embodiment of the present invention, the fluid vehicle is comprised in a gel having a melting temperature above ambient temperature and below 37°C (body temperature), which gel optionally comprises at least one of NaCl (aq), hyaluronic acid, PEG, propylene glycole alginate (PGA), titanium peroxy gel, methyl cellulose, carbomethyl cellulose, dextran, a high viscous polymeric gel, or a protein solution. This gel may be particularly useful in view of the fact that the gel and its containing particles will be easy to e.g. inject into a human or animal body when it is in a gel solid state at ambient temperature, but at the same time the gel becomes liquid at a normal body temperature making it readily and rapidly resorbable. Examples of such gels having that range of melting temperature may e.g. comprise hyaluronic acid in the right concentration for that gel to dissolve when being injected into a human or animal body.

There are different possible ways for bringing the particles for use according to the invention into contact with an intended infected site in a human or animal body. According to one specific embodiment of the present invention there is provided an injectable suspension comprising the particles of microstructure which particles are mixed with a fluid vehicle chosen from the group consisting of NaCl (aq), hyaluronic acid, PEG, titanium peroxy gel, methyl cellulose, carbomethyl as a medicament to be for use cellulose, dextran, a high viscous polymeric gel, and a protein solution, or a combination thereof, brought into contact with the at least one infected site by filling a cavity in the human or animal body, which cavity is present in the human or animal body or made by a surgical operation.

According to another specific embodiment of the present invention, there is provided an injectable suspension according to claim 1 wherein the particles of microstructure or the injectable suspension thereof are additionally treated in vivo by UV radiation with a wavelength λ of 200-500 nm. This is sometimes desirable due to the fact that it is hence possible to enhance the anti-inflammatory and/or antibacterial effect, as the UV radiation generates radicals. A specific suitable wave-length range of the UV radiation in many applications is 250-350 nm.

According to another specific embodiment, the cavity mentioned above is subsequently closed by a sealing composition. This sealing composition is e.g. fibrin glue, a membrane, e.g. of a polymer material, or collagen.

In accordance with one specific embodiment according to the present invention the injectable suspension comprising the particles of microstructure for use according to the invention is brought into contact with the at least one infected site by injecting the suspension into the human or animal body. Furthermore, according to one specific embodiment the particles of microstructure or the injectable suspension thereof are brought into contact with the at least one infected site at regular intervals to maintain the anti-inflammatory and/or antibacterial effect. Examples of specific conditions are periodontitis, periimplantitis, and osteitis.

The particles of microstructure or the injectable suspension used according to the present invention may also be of interest to inject into or insert to non-inflamed and/or non-infected sites of a human or animal body for different reasons, e.g. into specific parts or organs of a human or animal body in vivo or in vitro. Such parts or organs may e.g. be the intestine, liver, spleen, pancreas or e.g. the kidneys. One example of use of the particles of microstructure or the injectable suspension according to the present invention are as carriers of medicaments to specific parts of the human or animal body, where the particles either work just as a carriers or as active medicaments in combination with the other medicaments at the site intended to be contacted.

As mentioned before, the present invention includes an injectable suspension comprising
- particles of microstructure comprising titanium, titanium alloy, at least one type of titanium oxide or a combination thereof, and having a surface with at least a substantial part consisting of at least one type of titanium oxide, wherein the particles of microstructure have an average length from one side to the opposite side, through a geometrical centre, of < 1 mm; and
- a fluid vehicle,
as a medicament for alleviating and/or eliminating inflammatory and/or bacterial condition and/or promoting regeneration of tissue at a site of implantation.

Different specific embodiments of an injectable suspension are disclosed above with reference to the specific embodiments of the particular for use according to the present invention, wherein e.g. the average size of the particles varies, the shape of the particles of microstructure varies and the fluid vehicle could according to one specific embodiment be chosen from different type of forms, etc. Moreover, it should be understood that the suspension is possibly in the form of a paste, and further-more the vehicle may be a gel. In these cases, in comparison to a liquid suspension, the particles for use according to the present invention can be larger in size and still be evenly dispersed.

It is possible to combine the particles for use according to the present invention with more than just a fluid vehicle in the suspension. There could be different purposes for mixing such other substances into the injectable suspension. Such substances could e.g. be biologically active. Specific examples are antibiotics, factors promoting tissue growth or regeneration, or a combination thereof, e.g. bone morphogenic factor, fibroblast growth factor, andronate, alfa-keto glutarate, simvastatin, gentamicin or synthetic type I collagen. Other possible examples are at least one active enamel substance, which active enamel substance is enamel matrix, enamel matrix derivatives or enamel matrix proteins or combinations thereof, e.g. Emdogain®. Therefore, according to one embodiment of the present invention, there is provide an injectable suspension for use according to the invention, in which suspension at least one other substance has been admixed, the at least one other substance being chosen from the group consisting of antibiotics, factors promoting tissue growth and factors promoting tissue regeneration.

There are different ways of binding substances to, or modify, the particles for use according to the present invention. The physical and chemical surface modification methods can be categorised into three different types, the noncovalent coatings, the covalently attached coatings and modifications of the original surface.

The methods used for noncovalent coatings are preferably solvent coatings, surface-active additives or vapor deposition of carbons and metals, in which the latter one some covalent reaction may occur.

The preferred methods for covalently attached coatings are RFGD plasma deposition, in this case at low-pressure ionized gas environments typically at about ambient temperature, other plasma gas processes, gas-phase deposition, as chemical vapour deposition (CVD), chemical grafting and biological modification (biomolecule immobilization).

The methods for modifications of the original surface are preferably ion exchange, by chemical reactions, like non-specific oxidation, and conversion coatings.

It is also possible to treat the particles by different methods before they e.g. are brought into the suspension to improve some properties, if important. One example is to increase the specific surface area of the individual particles, which is possible to do by etching the particles. The increase of specific surface area could be of interest due to the fact that this is a measurement of the anti-inflammatory an/or antibacterial effects. This etching treatment could be performed by treating the particles with e.g. at least one fluoric compound, hydrochloric acid, sulphuric acid, phosphorous acid, a peroxide compound chosen from the group consisting of hydrogen peroxide (H₂O₂) and organic peroxides, or oxalic acid, or a combination thereof, or by dry etching with fluorinated or chlorinated gases. The fluoric compound is e.g. any type of fluoric acid, hydrofluoric acid in combination with nitric acid, ammonium fluoride, ammonium bifluoride (also in combination with nitric acid), or hydrogen fluoride (HF). Examples of concentrations of the chemicals are e.g. from 0.05 to 1.0% for fluoride acids, from 0.5 to 30.0% for hydrogen peroxides and from 0.2 to 20.0% for oxalic acids. Therefore, according to one specific embodiment of the present invention, the particles for use are pretreated with at least one chemical, the at least one chemical being chosen from the group consisting of at least one fluoric compound, hydrochloric acid, sulphuric acid, phosphorous acid, a peroxide compound chosen from the group consisting of hydrogen peroxide (H₂O₂) and organic peroxides, or oxalic acid, or a combination thereof, or are pretreated by dry etching with fluorinated or chlorinated gases.

Other possible treatments of the particles are oxidation, performed by heat treatment in oxidising atmosphere at temperatures between 20 and 1000°C and/or by an electrochemical procedure. The electrochemical procedure is possibly performed by anodical oxidation using spark erosion. This could also be performed to increase the surface area. The spark erosion procedure could be used to achieve beneficial properties. The surface is first exposed to spark erosion and particles are thereafter produced by a mechanical procedure, like shaving, grinding or filing.

Other treatment examples are heat treatment in inert atmosphere or vacuum at a temperature of 500°C or above, but in the case of titanium or titanium alloy particles below the melting point of the titanium or the titanium alloy, respectively.

Possible treatments could also have aesthetical purposes, which could be the case with some dental applications. Therefore titanium or titanium alloy particles could be oxidised to an extent where there is provided a titanium oxide layer on their surfaces with a substantial thickness of at least 500 nm. These particles are thereby made yellowish or whitish, which could be beneficial for some dental applications.

With the present invention the use of the particles is also disclosed. According to one specific embodiment there is provided use of particles of microstructure comprising titanium, titanium alloy, at least one type of titanium oxide or a combination thereof, and having a surface with at least a substantial part consisting of at least one type of titanium oxide, for the manufacture of a medicament in the form of an injectable suspension comprising the particles of microstructure and a fluid vehicle, which medicament is capable of alleviating and/or eliminating an inflammatory and/or bacterial condition and/or promoting regeneration of tissue at a site of implantation.

Specific embodiments in relation to the use of the particles according to the invention, for the manufacture of a medicament in the form of an injectable suspension is disclosed above with different specific embodiments referring to the injectable suspension for use according to the present invention. According to one aspect, there is e.g. provided use of particles of microstructure comprising titanium, titanium alloy, at least one type of titanium oxide or a combination thereof, and having a surface with at least a substantial part consisting of at least one type of titanium oxide, for the manufacture of a medicament in the form of an injectable suspension comprising the particles of microstructure and a fluid vehicle, which medicament is capable of alleviating and/or eliminating an inflammatory and/or bacterial condition and/or promoting regeneration of tissue at a site of implantation, wherein the fluid vehicle is chosen from the group consisting of NaCl (aq), hyaluronic acid, PEG, propylene glycole alginate (PGA), titanium peroxy gel, methyl cellulose, carbomethyl cellulose, dextran, a high viscous polymeric gel, and a protein solution, or a combination thereof. Another example is when the fluid vehicle is comprised in a gel having a melting temperature above ambient temperature and below 37°C (body temperature), which gel optionally comprises at least one of NaCl (aq), hyaluronic acid, PEG, propylene glycole alginate (PGA), titanium peroxy gel, methyl cellulose, carbomethyl cellulose, dextran, a high viscous polymeric gel, or a protein solution. Specific examples of the conditions are e.g. periodontitis, periimplantitis, and osteitis.

### Conclusions

Due to the present invention, wherein particles of microstructure are used to treat inflammatory and/or bacterial conditions, an effective way for treating these conditions is provided. Moreover, the material (particles) for use according to the invention exhibits an enhanced anti-inflammatory and/or antibacterial effect in relation to other materials according to state of the art. Moreover, in addition to the enhanced properties due to the structure in terms of size and shape, as well as the chemical composition of course, there are also clear practical benefits of the use of an injectable suspension according to the present invention being disclosed. Due to the injectable suspension as well as the particles for use according to the invention there is provided an effective method to bring an anti-inflammatory and/or antibacterial medicament into contact with an infected site, wherever located in a human or animal body.

### References

1. Sittig C, Textor M, Spencer ND, Wieland M, Vallotton PH. Surface characterization of implant materials c.p. Ti, Ti-6Al-7Nb and Ti-6Al-4V with different pretreatments. J Mater Sci Mater Med 1999;10(1):35-46.
2. Suzuki R, Muyco J, McKittrick J, Frangos JA. Reactive oxygen species inhibited by titanium oxide coatings. J Biomed Mater Res A 2003;66(2):396-402.
3. Scharnweber D, Beutner R, Rossler S, Worch H. Electrochemical behavior of titanium-based materials - are there relations to biocompatibility? J Mater Sci Mater Med 2002;13(12):1215-20.
4. Zhang F, Zheng Z, Chen Y, Liu X, Chen A, Jiang Z. In vivo investigation of blood compatibility of titanium oxide films. J Biomed Mater Res 1998;42(1): 128-33.
5. Tengvall P, Lundstrom I, Sjoqvist L, Elwing H, Bjursten LM. Titanium-hydrogen peroxide interaction: model studies of the influence of the inflammatory response on titanium implants. Biomaterials 1989;10(3):166-75.
6. Tengvall P, Elwing H, Sjoqvist L, Lundstrom I, Bjursten LM. Interaction between hydrogen peroxide and titanium: a possible role in the biocompatibility of titanium. Biomaterials 1989;10(2):118-20.
7. Ragai J. Trapped radicals in titania gels. Nature 1987;325(6106):703-5.
8. Wick PK, Kissner R, Koppenol WH. Kinetics evidence for a complex between peroxynitrous acid and titanium(IV). Inorg Chem 2004;43(16):4805-7.
9. Tengvall P, Lundstrom I. Physico-chemical considerations of titanium as a biomaterial. Clin Mater 1992;9(2):115-34.
10. Sundgren J-E, Bodo P, Lundstrom I. Auger electron spectroscopic studies of the interface between human tissue and implants of titanium and stainless steel. J Colloid Interface Sci 1986;110(1):9-20.
11. Guang Pu Xue Yu Guang Pu Fen Xi. 2002 Oct;22(5):783-6. Study on nanophase anatase-rutile transition with Raman spectrum.

## Claims

1. An injectable suspension comprising
- particles of microstructure comprising titanium, titanium alloy, at least one type of titanium oxide or a combination thereof, and having a surface with at least a substantial part consisting of at least one type of titanium oxide, wherein the particles of microstructure have an average length from one side to the opposite side, through a geometrical centre, of < 1 mm; and
- a fluid vehicle,
for use as a medicament for alleviating and/or eliminating an inflammatory and/or bacterial condition and/or promoting regeneration of tissue at a site of implantation.

2. The injectable suspension for use according to claim 1, for use in the treatment of an inflammatory and/or bacterial condition chosen from the group consisting of periodontitis, periimplantitis, and osteitis.

3. The injectable suspension for use according to claim 1 or 2, wherein the particles of microstructure have an average length from one side to the opposite side, through a geometrical centre, of < 0.5 mm.

4. The injectable suspension for use according to any of claims 1-3, wherein the particles of microstructure have an average length from one side to the opposite side, through a geometrical centre, of < 0.2 mm.

5. The injectable suspension for use according to any of claims 1-4, wherein the particles of microstructure have an average length from one side to the opposite side, through a geometrical centre, of < 0.1 mm.

6. The injectable suspension for use according to any of the preceding claims, wherein the particles of microstructure have an average length from one side to the opposite side, through a geometrical centre, in the range of 0.01-0.1 mm.

7. The injectable suspension for use according to any of the preceding claims, wherein the particles of microstructure have an average length from one side to the opposite side, through a geometrical centre, of < 0.01 mm.

8. The injectable suspension for use according to any of the preceding claims, wherein the particles of microstructure are in the shape of spheres, spikes, flakes, chips or similar or combinations thereof.

9. The injectable suspension for use according to any of the preceding claims, wherein the fluid vehicle is chosen from the group consisting of NaCl (aq), hyaluronic acid, PEG, propylene glycole alginate (PGA), titanium peroxy gel, methyl cellulose, carbomethyl cellulose, dextran, a high viscous polymeric gel, and a protein solution, or a combination thereof.

10. The injectable suspension for use according to any of the preceding claims, in which injectable suspension at least one other substance has been admixed, the at least one other substance being chosen from the group consisting of antibiotics, factors promoting tissue growth and factors promoting tissue regeneration.

11. The injectable suspension for use according to claim 11, wherein the at least one other substance is chosen from the group consisting of bone morphogenic factor, fibroblast growth factor, andronate, alfa-keto glutarate, simvastatin, gentamicin, synthetic type I collagen and at least one active enamel substance, which active enamel substance is enamel matrix or enamel matrix proteins or combinations thereof.

12. The injectable suspension for use according to any of the preceding claims, in which injectable suspension the particles are pretreated with at least one chemical, the at least one chemical being chosen from the group consisting of at least one fluoride acid, hydrochloric acid, sulphuric acid, phosphorous acid, a peroxide compound chosen from the group consisting of hydrogen peroxide (H₂O₂) and organic peroxides and oxalic acid, or are pretreated by dry etching with fluorinated or chlorinated gases.

## Patentansprüche

1. Injektionssuspension, umfassend
- Teilchen mit Mikrostruktur, die Titan, Titanlegierung, mindestens eine Art von Titanoxid oder eine Kombination davon umfassen und eine Oberfläche aufweisen, die mindestens zu einem wesentlichen Teil aus mindestens einer Art von Titanoxid besteht, wobei die Teilchen mit Mikrostruktur eine durchschnittliche Länge von einer Seite zur gegenüberliegenden Seite durch einen geometrischen Mittelpunkt von < 1 mm aufweisen; und
- ein fließfähiges Vehikel,
zur Verwendung als Medikament zur Milderung und/oder Eliminierung eines entzündlichen und/oder bakteriellen Leidens und/oder zur Förderung der Geweberegeneration an einem Implantationsort.

2. Injektionssuspension zur Verwendung nach Anspruch 1 zur Verwendung bei der Behandlung eines entzündlichen und/oder bakteriellen Leidens aus der Gruppe bestehend aus Periodontitis, Periimplantitis und Osteitis.

3. Injektionssuspension zur Verwendung nach Anspruch 1 oder 2, wobei die Teilchen mit Mikrostruktur eine durchschnittliche Länge von einer Seite zur gegenüberliegenden Seite durch einen geometrischen Mittelpunkt von < 0,5 mm aufweisen.

4. Injektionssuspension zur Verwendung nach einem der Ansprüche 1-3, wobei die Teilchen mit Mikrostruktur eine durchschnittliche Länge von einer Seite zur gegenüberliegenden Seite durch einen geometrischen Mittelpunkt von < 0,2 mm aufweisen.

5. Injektionssuspension zur Verwendung nach einem der Ansprüche 1-4, wobei die Teilchen mit Mikrostruktur eine durchschnittliche Länge von einer Seite zur gegenüberliegenden Seite durch einen geometrischen Mittelpunkt von < 0,1 mm aufweisen.

6. Injektionssuspension zur Verwendung nach einem der vorhergehenden Ansprüche, wobei die Teilchen mit Mikrostruktur eine durchschnittliche Länge von einer Seite zur gegenüberliegenden Seite durch einen geometrischen Mittelpunkt im Bereich von 0,01-0,1 mm aufweisen.

7. Injektionssuspension zur Verwendung nach einem der vorhergehenden Ansprüche, wobei die Teilchen mit Mikrostruktur eine durchschnittliche Länge von einer Seite zur gegenüberliegenden Seite durch einen geometrischen Mittelpunkt von < 0,01 mm aufweisen.

8. Injektionssuspension zur Verwendung nach einem der vorhergehenden Ansprüche, wobei die Teilchen mit Mikrostruktur in Form von Kugeln, Nadeln, Schuppen, Schnittstellen oder dergleichen oder Kombinationen davon vorliegen.

9. Injektionssuspension zur Verwendung nach einem der vorhergehenden Ansprüche, wobei das fließfähige Vehikel aus der Gruppe bestehend aus NaCl (aq), Hyaluronsäure, PEG, Propylenglykolalginat (PGA), Titanperoxygel, Methylcellulose, Carbomethylcellulose, Dextran, einem hochviskosen Polymergel und einer Proteinlösung oder einer Kombination davon ausgewählt ist.

10. Injektionssuspension zur Verwendung nach einem der vorhergehenden Ansprüche, wobei der Injektionssuspension mindestens eine andere Substanz beigemischt ist, wobei die mindestens eine andere Substanz aus der Gruppe bestehend aus Antibiotika, gewebewachstumsfördernden Faktoren und geweberegenerationsfördernden Faktoren ausgewählt ist.

11. Injektionssuspension zur Verwendung nach Anspruch 11, wobei die mindestens eine andere Substanz aus der Gruppe bestehend aus Knochenmorphogenesefaktor, Fibroblasten-Wachstumsfaktor, Andronat, alpha-Ketoglutarat, Simvastatin, Gentamicin, synthetischem Kollagen Typ I und mindestens einer aktiven Zahnschmelzsubstanz ausgewählt ist, wobei es sich bei der aktiven Zahnschmelzsubstanz um Zahnschmelzmatrix oder Zahnschmelzmatrixproteine oder Kombinationen davon handelt.

12. Injektionssuspension zur Verwendung nach einem der vorhergehenden Ansprüche, wobei in der Injektionssuspension die Teilchen mit mindestens einer Chemikalie vorbehandelt sind, wobei die mindestens eine Chemikalie aus der Gruppe bestehend aus mindestens einer Fluoridsäure, Salzsäure, Schwefelsäure, Phosphorsäure, einer Peroxidverbindung aus der Gruppe bestehend aus Wasserstoffperoxid (H₂O₂) und organischen Peroxiden und Oxalsäure ausgewählt ist, oder durch Trockenätzen mit fluorierten oder chlorierten Gasen vorbehandelt sind.

## Revendications

1. Suspension pour injection comprenant :
- des particules de microstructure comprenant du titane, un alliage de titane, au moins un type d'oxyde de titane ou l'une de leurs combinaisons, et présentant une surface dont au moins une portion substantielle est constituée d'au moins un type d'oxyde de titane, où les particules de microstructure présentent une longueur moyenne de l'un des côtés au côté opposé, via un centre géométrique, < 1 mm ; et
- un vecteur liquide,
pour emploi en tant que médicament destiné au soulagement et/ou à l'élimination d'un état inflammatoire et/ou bactérien et/ou à la promotion de la régénération des tissus au niveau d'un site d'implantation.

2. Suspension pour injection pour emploi conforme à la revendication 1, pour emploi dans le traitement d'un état inflammatoire et/bactérien choisi dans le groupe constitué par la parodontite, la péri-implantite et l'ostéite.

3. Suspension pour injection pour emploi conforme à la revendication 1 ou 2, où les particules de microstructure présentent une longueur moyenne de l'un des côtés au côté opposé, via un centre géométrique, < 0,5 mm.

4. Suspension pour injection pour emploi conforme à l'une quelconque des revendications 1 à 3, où les particules de microstructure présentent une longueur moyenne de l'un des côtés au côté opposé, via un centre géométrique, < 0,2 mm.

5. Suspension pour injection pour emploi conforme à l'une quelconque des revendications 1 à 4, où les particules de microstructure présentent une longueur moyenne de l'un des côtés au côté opposé, via un centre géométrique, < 0,1 mm.

6. Suspension pour injection pour emploi conforme à l'une quelconque des revendications précédentes, où les particules de microstructure présentent une longueur moyenne de l'un des côtés au côté opposé, via un centre géométrique, comprise entre 0,01 et 0,1 mm.

7. Suspension pour injection pour emploi conforme à l'une quelconque des revendications précédentes, où les particules de microstructure présentent une longueur moyenne de l'un des côtés au côté opposé, via un centre géométrique, < 0,01 mm.

8. Suspension pour injection pour emploi conforme à l'une quelconque des revendications précédentes, où les particules de microstructure prennent la forme de sphères, de pics, de flocons, de copeaux, etc., ou de leurs combinaisons.

9. Suspension pour injection pour emploi conforme à l'une quelconque des revendications précédentes, où le vecteur liquide est choisi dans le groupe constitué par les suivants : NaCl (aq), acide hyaluronique, PEG, alginate de propylène glycol (PGA), peroxygel de titane, méthylcellulose, carbométhylcellulose, dextrane, gel polymère de viscosité élevée et solution protéinique, ou l'une de leurs combinaisons.

10. Suspension pour injection pour emploi conforme à l'une quelconque des revendications précédentes, où dans ladite suspension pour injection, au moins une autre substance a été ajoutée et mélangée, la ou lesdites autres substances étant choisies dans le groupe constitué par les antibiotiques, les facteurs favorisant la croissance tissulaire et les facteurs favorisant la régénération tissulaire.

11. Suspension pour injection pour emploi conforme à la revendication 11, où la ou lesdites autres substances sont choisies dans le groupe constitué par les suivantes : facteur morphogénétique osseux, facteur de croissance des fibroblastes, andronate, alphacétoglutarate, simvastatine, gentamicine, collagène de type I de synthèse et au moins une substance émaillée active, ladite substance émaillée active étant une matrice émaillée, des protéines de matrice émaillée ou l'une de leurs combinaisons.

12. Suspension pour injection pour emploi conforme à l'une quelconque des revendications précédentes, où dans ladite suspension pour injection, les particules sont prétraitées par au moins une substance chimique, la ou lesdites substances chimiques étant choisies dans le groupe constitué par les suivantes : au moins un acide fluoré, acide chlorhydrique, acide sulfurique, acide phosphoreux, peroxyde choisi dans le groupe constitué par le peroxyde d'hydrogène (H₂O₂) et les peroxydes organiques, et acide oxalique, ou sont prétraitées par gravure à sec par des gaz fluorés ou chlorés.
